(19) Europäisches Patentamt
European Patent Office
Office européen des brevets

(11) Numéro de publication : **0 578 560 A2**

# (12) DEMANDE DE BREVET EUROPEEN

(21) Numéro de dépôt : **93401749.2**

(22) Date de dépôt : **06.07.93**

(51) Int. Cl.[5] : **C07D 211/16,** A61K 31/395, C07D 451/02, C07D 223/14, C07D 217/06, C07D 209/02

(30) Priorité : **08.07.92 FR 9208462**

(43) Date de publication de la demande : **12.01.94 Bulletin 94/02**

(84) Etats contractants désignés : **AT BE CH DE DK ES FR GB GR IE IT LI LU MC NL PT SE**

(71) Demandeur : **CENTRE D'ETUDES EXPERIMENTALES ET CLINIQUES DE PHYSIO-BIOLOGIE, DE PHARMACOLOGIE ET D'EUTONOLOGIE (CEPBEPE)**
**78, rue de la Convention**
**F-75015 Paris (FR)**

(72) Inventeur : **Laborit, Henri**
**93 rue de la Santé**
**F-75013 Paris (FR)**
Inventeur : **Zerbib, Robert**
**4 avenue de la République**
**F-92130 Issy-les-Moulineaux (FR)**
Inventeur : **Dostert, Philippe**
**10 Place des Vosges**
**F-75004 Paris (FR)**

(74) Mandataire : **Bloch, Gérard**
**2, square de l'Avenue du Bois**
**F-75116 Paris (FR)**

## (54) Dérivés amido-alcools comme agents psychotropes.

(57) Dérivés cycliques représentés par la formule générale

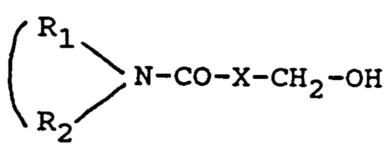

(I)

dans laquelle le système cyclique

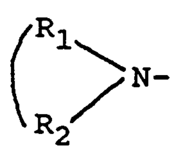

représente pipéridine, nor-tropane, 3-azabicyclo[3,2,2]nonane, 6-azabicyclo [3,2,1]octane, ou tétrahydro-1,2,3,4-isoquinoline, éventuellement substitué par un ou plusieurs groupes alkyle, et

X représente le résidu $CH_2$-$CH_2$ ou CH=CH (trans)

Utilisation de ces dérivés pour le traitement des affections neurologiques et psychiatriques.

EP 0 578 560 A2

La présente invention concerne une nouvelle famille de composés chimiques dont les membres se distinguent par leur action psychotrope.

Ils sont représentés par la formule générale (I)

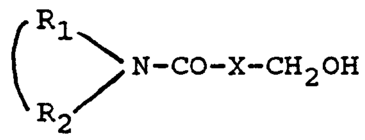

(I)

dans laquelle le système cyclique

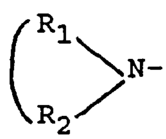

représente pipéridine, nor-tropane, 3-azabicyclo[3,2,2]nonane, 6-azabicyclo[3,2,1]octane, ou tétrahydro-1,2,3,4-isoquinoline, éventuellement substitué par un ou plusieurs groupes alkyle et X représente le résidu $CH_2$-$CH_2$ ou CH = CH (trans)

La structure de certains de ces composés est indiquée sur le tableau 1 et décrite dans la description.

Les composés selon l'invention sont obtenus par deux voies différentes selon que :

a) - X est $CH_2$-$CH_2$

dans ce cas les composés I sont obtenus par réaction d'une amine

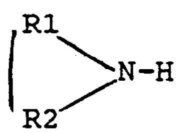

(A) avec la γ-butyrolactone à reflux dans un solvant tel que le benzène ou le toluène

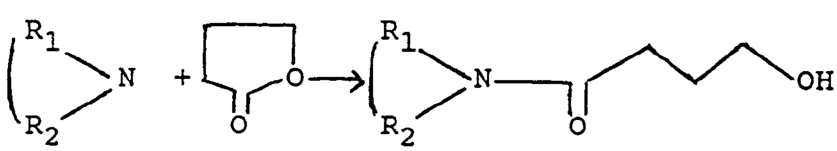

Le composé selon l'invention pour lequel

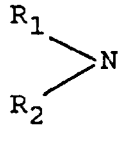

est le cycle pipéridine a été obtenu précédemment (Bull. Chem. Soc. Jpn. 62:3138-3142, 1989) par réaction de la pipéridine avec la γ -butyrolactone sous haute pression. A notre connaissance les autres composés sont nouveaux.

b) - X est CH=CH (trans)

dans ce cas les composés I sont obtenus par réduction de l'acide II. Dans un premier temps II est mis à réagir avec un chloroformiate, tel le chloroformiate d'éthyle, en présence d'un accepteur de proton telle la triéthylamine (TEA) puis la réduction a lieu par addition de borohydrure de sodium :

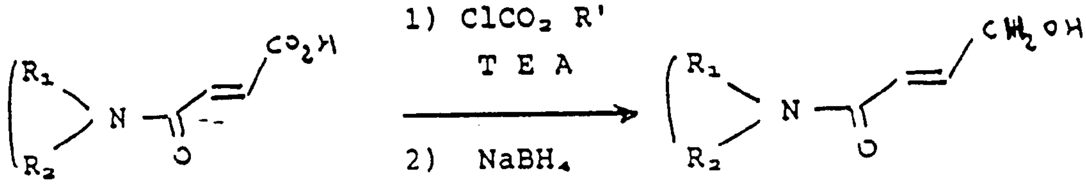


II I

L'acide II est obtenu par hydrolyse basique par la potasse ou la soude méthanolique de l'ester correspondant III, dans lequel $R_3$ représente un groupe alkyle préférentiellement éthyle, selon le schéma :

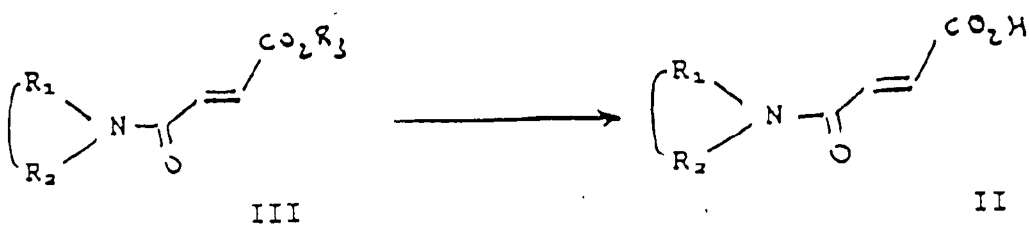

III II

L'ester III est obtenu par réaction d'une amine A avec l'hémi-chlorure d'un hémi-ester de l'acide fumarique IV, obtenu selon J. Chem. Soc., p. 1501 (1951), dans lequel le groupe $R_3$ a la même signification que précédemment, en présence d'un accepteur de proton telle la trièthylamine :

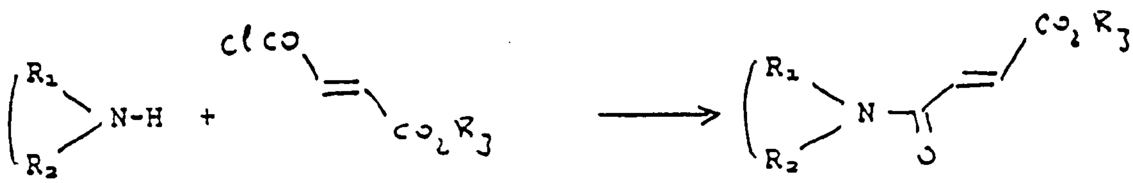

A IV III

La synthèse des composés suivants est donnée à titre d'exemple :

<u>Exemple 1 - Composés avec A = $CH_2$-$CH_2$</u>

N-(γ-hydroxybutyryl) pipéridine (FCE 26099)

La solution de 8,6 g (0,1 mole) de γ butyrolactone et 12,8 g (0,15 mole) de piperidine dans 75 ml de benzène anhydre est portée à reflux pendant 5 heures. La solution est évaporée à sec sous vide et le résidu est purifié sur colonne de silice par flash-chromatographie en utilisant comme éluant un mélange de $CHCl_3$/MeOH/$NH_4OH$ (30 %) : 90/10/1.

On obtient ainsi 13,5 g de N-( γ -hydroxybutyryl) pipéridine (rendement 78,9 %) ; point de fusion : 31-33 degrés C ; la littérature (Bull. Chem. Soc. Jpn. 62:3138-3142, 1989) décrit ce produit comme une huile.

De manière analogue ont été obtenus :

- à partir du nortropane, le dérivé :
  N-( γ -hydroxybutyryl) nortropane (FCE 26630); rendement 57 %; $n_D^{20}$ = 1,5164; NMR, ($CDCl_3$ : 1.4-2.0 (12H, m), 2,3-2,6 (2H, m, CO-$CH_2$), 3,65 (2H, t, $CH_2OH$), 4,12 (1H, m), 4,62 (1H, m).
- partir du 3-azabicyclo[3,2,2]nonane, le dérivé :
  3-(γ-hydroxybutyryl)-3-azabicyclo[3,2,2]nonane (FCE 26857); rendement 74,8 %, $n_D^{20}$ = 1,5240; NMR, ($CDCl_3$) : 1,5-2,1 (12H, m), 2,5 (2H, t, CO<u>$CH_2$</u>), 3,21 (1H, t, OH), 3,55 (2H, d, N<u>$CH_2$</u>), 3,65 (2H, t, <u>$CH_2$</u>OH), 3,70 (2H, d, N-<u>$CH_2$</u>).
- à partir du 1,3,3-triméthyl-6-azabicyclo[3,2,1] octane, le dérivé :
  6- ( γ hydroxybutyryl)-1,3,3,-triméthyl-6-azabicyclo [3,2,1]octane (FCE 26858), rendement 78,3 %; $n_D^{20}$ = 1,5020 ; NMR, ($CDCl_3$ : 0,9 (6H, s, C(<u>$CH_3$</u>)2), 1,05 (3H, s, <u>$CH_3$</u>-C), 1,2-2,0 (8H, m), 2,2-2,6 (2H, m,

CO$\underline{CH_2}$), 3,0-3,45 (2H, m, $\underline{CH_2}$N), 2,55-2,75 (3H, m, $\underline{CH2OH}$), 4,1 + 4,45 (1H, m, N-$\underline{CH}$ anti+syn).

- partir de la tétrahydro-1,2,3,4-isoquinoline, le dérivé : 1,2,3,4-tétrahydro-2(γ -hydroxybutyryl)isoquinoline (FCE 27407); rendement 72,5 %, $n_D^{20}$ = 1,5681; NMR, ($CDCl_3$) : 1,92 (2H, m, $COCH_2\underline{CH_2}$), 2,55 (2H, m, $CO\underline{CH_2}CH_2$), 2,88 (2H, m, N-$CH_2$-$\underline{CH_2}$-Arom.), 3,2 (1H, b.s., $\underline{OH}$), 3,70 (2H, m, N-$\underline{CH_2}$-$CH_2$-Arom.), 3,82 (2H, t, $\underline{CH_2}$OH), 4,66 (2H, d, N-$\underline{CH_2}$-Arom.), 7,1 (4H, m, Arom.).

Exemple 2 - composés avec A = CH=CH (trans)

6- (γ-hydroxycrotonyl)-1,2,3-triméthyl-6-azabicyclo [3,2,1] octane (FCE 27363)

A la solution, refroidie à 0 degré C, de 11 g (0,0437 mole) de l'acide trans-β-[N-1,2,3-triméthyl-6-azabicyclo[3,2,1]octane) carboxamide]acrylique et de 6 g (0,06 mole) de triéthylamine dans 250 ml de diglyme, est ajoutée en 45 minutes la solution de 4,17 ml (0,0437 mole) de chloroformiate d'éthyle dans 50 ml de diglyme. Après agitation à 0 degré C pendant 2 heures, 3,3 g de borohydrure de sodium sont ajoutés en 1 heure en maintenant la température à 0 degré C. Après 90 minutes à cette température, 250 ml d'eau froide sont additionnés en maintenant la température entre 0 et 5 degrés C; l'agitation est continuée à 15 degrés C pendant 16 heures. La solution est concentrée sous vide et après addition de 60 ml d'eau, la solution aqueuse est extraite quatre fois par du chlorure de méthylène. Après séchage et évaporation sous vide, le résidu est purifié par flash-chromatographie sur silice avec un mélange acétate d'éthyle/méthanol : 90/10 comme éluant.

On obtient 3,85 g (37 %) de ( γ -hydroxycrotonyl)-1,2,3-triméthyl-6-azabicyclo[3,2,1]octane, point de fusion 72-75 degrés C.

Acide trans- β-[N-(1,2,3-triméthyl-6-azabicyclo[3,2,1] octane)-carboxamide]acrylique

La solution de 36 g (0,129 mole) d'ester éthylique de l'acide trans-β-[(1,2,3-triméthyl-6-azabicyclo[3,2,1] octane)carboxamide acrylique dans 390 ml de potasse N/2 dans le méthanol est agitée pendant 3 heures à température ambiante. La solution est évaporée à sec, puis est acidifiée à froid, après addition d'eau, avec une solution froide de HCl 1N. Après extraction à l'acétate d'éthyle, la phase organique est lavée à l'eau, séchée puis évaporée à sec. Après trituration du résidu en presence d'isopropanol et filtration, on obtient 24 g (rendement 74 %) d'acide trans-β- [N-(1,2,3-triméthyl-6-azabicyclo[3,2,1]octane)carboxamide]acrylique ; point de fusion 161-163 degrés C.

Ester étylique de l'acide trans-β-[N-[1,2,3-triméthyl-6-azabicyclo[3,2,1]octane)carboxamide]acrylique

A la solution de 26,2 ml (0,154 mole) de 1,2,3-triméthyl-6-azabicyclo[3,2,1]octane et 22,4 ml de triéthylamine (0,16 mole) dans 250 ml de chlorure de méthylène refroidie à 5-10 degrés C, la solution de 25 g (0,154 mole) de chlorure de l'acide trans-β - carbethoxyacrylique dans 70 ml de chlorure de méthylène est ajoutée en 1 heure en maintenant la température entre 5 et 10 degrés C. A la fin de l'addition, la solution est agitée à température ambiante pendant 90 minutes, lavée à l'eau, séchée et évaporée à sec. Le résidu est purifié par flash-chromatographie sur silice en utilisant un mélange acétate d'éthyle/hexane : 1/1 comme éluant. On obtient 38,5 g (89 %) d'ester éthylique de l'acide trans-β-[N-[(1,2,3-triméthyl-6-azabicyclo [3,2,1]octane)carboxamide]acrylique ; point de fusion 32-38 degrés C.

Les composés selon l'invention présentent tous une action psychotrope notamment des activités mnémotoniques, antiparkinsonienne, antidépressives et antipsychotique.

A titre d'exemple sont indiqués les résultats d'études expérimentales réalisées sur le composé FCE 26858.

1) ACTIVITE MNEMOTONIOUE

L'activité mnémotonique a été mise en évidence par le test de l'antagonisme de l'amnésie induite par la scopolamine sur un évitement passif et sur un évitement actif, le "Wall Jump Test" chez la souris. Ces activités ont été comparées à celles de l'ANIRACETAM, molécule active dans ce type de test (Moos W.H. et al. Medicinal Research Reviews, vol. 8, n 3 : 353-391, 1988).

EVITEMENT PASSIF

Protocole experimental

**Animaux**

Les animaux utilisés sont des souris mâles OF1 d'un poids moyen de 30 grammes, divisées en lots de neuf animaux.

**Appareillage de l'évitement passif**

L'entraînement et le test de l'évitement passif ont été conduits avec deux cages en plexiglas blanc et noir. Le compartiment blanc (40 cm de long) est éclairé par une ampoule de 100 W ; le compartiment noir (30 cm de long) comporte un plancher électrifié composé de barreaux de 2 mm de diamètre, espaces d'un centimètre. Un courant constant de 1 mA est délivré (APELEX) dans les barreaux du compartiment noir. Une ouverture de 7 x 7 cm dans le mur séparant les deux moitiés de la cage peut être fermée par une porte guillotine coulissante.

**Méthode d'entraînement et de test**

Les souris sont divisées au hasard en lots de neuf animaux et sont soumises à une série d'entraînement au jour 1 et sont testées pour l'acquisition de l'évitement passif, 24 heures après. Le jour de l'entraînement, les souris sont placées individuellement dans le compartiment blanc éclairé, en face du compartiment noir.

Quand la souris est entrée dans le compartiment noir, la porte est fermée et un choc électrique de cinq secondes est administré. 24 heures après, chaque souris est de nouveau placée dans le compartiment blanc en face du compartiment noir.

Le temps de latence pour pénétrer dans le compartiment noir est mesuré pendant trois minutes. On attribue un temps de latence de 180 secondes aux souris ne pénétrant pas dans le compartiment noir.

**Drogues**

Le groupe témoin reçoit du NaCl 0,9 % en ip, sous un volume de 0,1 ml/10 g, 20 minutes avant l'entraînement.

Le groupe scopolamine reçoit du NaCl à 0,9 %, 15 minutes avant l'injection ip de scopolamine hydrobromide 0,3 mg/kg administrée 5 minutes avant l'entraînement.

Le FCE 26858 est administré 15 minutes avant la scopolamine aux doses de 0,0075, 0,015, 0,030, 0,060, 0,12 mmole/kg (1,8, 3,6, 7,2, 14,4 et 28,7 mg/kg ip).

L'ANIRACETAM est administré 15 minutes avant la scopolamine aux doses de 0,23, 0,32, 0,45, 0,63 et 0,88 mmole/kg (50, 70, 98, 137,2 et 192,1 mg/kg ip).

**Résultats**

Les résultats sont exprimés dans les figures 1 et 2. Les temps de latence sont comparés à l'aide du test du U de Mann et Whitney avec * pour $p < 0,05$ et ** pour $p < 0,01$.

Pour comparer les deux molécules nous avons calculé une $DE_{50}$ correspondant à la dose efficace qui antagonise complètement l'amnésie induite par la scopolamine chez 50 % des animaux.

Les figures 1 et 2 montrent que la scopolamine 0,3 mg/kg ip diminue le temps de latence pour pénéter dans le compartiment noir 24 heures après entraînement et que le FCE 26858 et l'ANIRACETAM s'opposent significativement à cette amnésie en fonction des doses. La courbe en U inversé des résultats est classique des produits actifs sur ce type de test.

Le calcul des $DE_{50}$ nous donne :

- pour le FCE 26858 : 7,6 mg/kg ip [3,8 - 15,3] 0,032 mmole/kg ip)
- pour l'ANIRACETAM : 78,3 mg/kg ip [59,8 - 102,4] 0,36 mmole/kg ip)

soit une activité dix fois plus importante du FCE 26858 par rapport à l'ANIRACETAM.

<u>ETUDE DU FCE 26858 ET DE L'ANIRACETAM SUR UN TEST D'EVITEMENT ACTIF : "WALL JUMP TEST"</u>

<u>Protocole expérimental</u>

**Animaux** :

L'expérimentation est réalisée sur des souris mâles d'un poids moyen de 30 grammes provenant des établissements IFFA CREDO (France). Les animaux sont laissés une semaine au laboratoire dans des conditions standards avant toute expérimentation.

**Matériel** :

Le matériel utilisé est une boîte en plexiglas noir (40 x 35 x 30) comportant un plancher composé de barreaux électrifiables de 2 mm de diamètre et espacé de 1 cm. Un courant de 0,35 mA est administré (24 V Power Supply, Campden Instrument LTD). Le compartiment peut être éclairé par une ampoule de 100 Watts. Les trois murs de la boîte sont recouverts d'un grillage plastifié permettant l'accrochage des animaux.

**Procédure** :

Au jour 1, l'animal est déposé dans la boîte pendant trente secondes pour permettre l'exploration ; à $t_{30}$, le signal lumineux est actionné et à $t_{35}$, le choc électrique est délivré. L'essai est interrompu quand l'animal saute sur un des murs grillagés avec un maximum de vingt secondes de choc.

Le deuxième essai est identique sauf que le temps d'exploration est supprimé. Dix essais sont effectués aux jours 1, 2, 3 et 8.

On note le temps moyen nécessaire à l'animal pour sauter sur un des murs grillagés et le nombre moyen d'évitement caractérisé par le saut sur un des murs grillagés avant le choc électrique sur les dix essais. On attribue à l'animal sautant sur un mur avant le choc électrique un score de zéro et un score de vingt-cinq pour l'animal ne sautant pas pendant toute la durée de l'essai (5 secondes de stimulus lumineux + 20 secondes de choc électrique).

**Drogues** :

Le FCE 26858 0,10 et 0,25 mmole/kg ip (24 et 60 mg/kg) et l'ANIRACETAM 0,46 mmole/kg ip (100 mg/kg) sont administrés trente minutes avant le test aux jours 1, 2, 3 et 8. Le groupe témoin reçoit du Nacl à 0,9 % en ip.

**Résultats** :

La comparaison statistique est réalisée à l'aide du test de Student avec $*$ $p < 0,05$ et $**$ $p < 0,01$.

Les figures 3 et 4 montrent que le FCE 26858 améliore significativement pour la dose de 0,25 mmole/kg ip les deux paramètres du "Wall Jump Test" mesurés aux jours 3 et 8. L'ANIRACETAM améliore significativement ces paramètres à la dose utilisée uniquement au jour 8.

**Conclusion**

Le FCE 26858 présente donc une activité mnémotonique intéressante et supérieure à celle de l'ANIRACETAM. Son utilisation sera indiquée dans les troubles mnésiques accompagnant le vieillissement, les traumatismes crâniens et les maladies neurodégénératives dont la maladie d'Alzheimer.

2) <u>ACTIVITE ANTIPARKINSONIENNE</u>

La maladie de Parkinson est caractérisée par une déficience de la composante dopaminergique des ganglions de la base, attribuée à une perte de neurones dans la substance noire. Le déficit en dopamine (DA) provoque des tremblements, une bradykinésie et une rigidité musculaire. Ainsi le but théorique du traitement de la maladie de Parkinson est de rétablir l'activité striatale en réduisant l'activité cholinergique ou en augmentant la fonction dopaminergique (The pharmacological basis of therapeutics, Goodman and Gilman VII edition, Mac Millan, 1985).

Or l'administration de FCE 26858 provoque de manière dose-dépendante une augmentation importante de la libération de DA dans le striatum de cerveau de souris, caractérisée par un taux d'acide homovanillique (HVA) élevé sans modification du taux de la DA laissant supposer que la synthèse de ce neuromédiateur est conservée. D'autre part, le FCE 26858 ne provoque jamais de stéréotypies.

Pour illustrer l'activité du FCE 26858 sur le système dopaminergique, nous rapportons ici son action à la dose de 0,25 mmole/kg (60 mg/kg) en injection intrapéritonéale (ip) sur la dopamine et ses métabolites du striatum de cerveau de souris en fonction du temps.

ETUDE DU FCE 26858 SUR LE TURNOVER DE LA DOPAMINE

Protocole expérimental

**Animaux**

Les animaux utilisés sont des souris mâles OF1 d'un poids moyen de 30 grammes, soumises au régime standard de laboratoire. Ces animaux sont répartis en lots de sept animaux :
- les lots témoins recevant une injection intrapéritonéale de soluté salé isotonique,
- les lots essais recevant du FCE 26858 0,25 mmole/kg ip (60 mg/kg).

Les animaux sont sacrifiés par décapitation 30, 60 et 120 minutes après l'administration des produits.

**Technique de dosage**

Les cerveaux sont rapidement prélevés et disséqués sur glace selon la méthode de Glowinsky et Iversen. Les striatums sont conservés ensuite à -70 degrés C jusqu'à analyse. Les échantillons sont pesés puis homogénéisés avec un Ultra-Turrax dans 450 µl d'acide perchlorique 0,4 N contenant 0,1 % EDTA et $NA_2S_2O_5$. Les homogénats sont centrifugés à 8 000 r.p.m. et à 4 degrés C pendant dix minutes. Le surnageant est ensuite amené à un pH de 5 par addition de 50 µl d'acétate de potassium 10 M. Les échantillons sont centrifugés à nouveau comme précédemment. Le surnageant est alors injecté directement dans l'H.P.L.C. selon la technique de Morier et Rips.

Le système H.P.L.C. utilisé est constitué d'une pompe Waters 6 000 A, d'un injecteur automatique réfrigéré à 4 degrés C CMA 200 Carnegie Médecine, d'un système à compression radicale, d'une colonne Radial PAK A ($C_{18}$ 0,8 cm i.d.x. 10 cm, 10 µm) et d'un detecteur électrochimique Metrohm 641. Le potentiel utilisé est + 0,8 V. La colonne est thermostatée à 30 degrés C et la vitesse d'élution est de 1 ml/min.

La phase mobile est constituée de $KH_2PO_4$ 0,1 M, de EDTA 0,1 M, d'acide heptane sulfonique 5 mM (Waters Pic B7) et de méthanol 15 % (V/V). Le pH de la phase mobile est ajusté à 4,10. Toutes les solutions sont préparées avec de l'eau purifiée avec le système Millipore Milli Q. La phase mobile est ensuite filtrée au travers de filtres Millipore 0,45 µm puis dégazée.

Les résultats sont comparés par le test du t de Student avec NS : non significatif ; * $p < 0,05$ ; ** $p < 0,01$ ; *** $p < 0,001$.

**Résultats**

La figure 5 montre que le FCE 26858 provoque une augmentation de l'HVA, une diminution du DOPAC (Acide 3-4-dihydroxyphénylacétique) en fonction du temps. Le taux de DA n'est pas modifié significativement.

Ces résultats indiquent une augmentation de la libération de DA avec conservation de la synthèse.

Nous avons étudié aussi l'action du FCE 26858 sur un modèle animal des tremblements caractéristiques de la maladie de Parkinson (Kaakkola S. et al. Pharmacology and Toxicology, Nr 67 : 95-100, 1990).

ACTION DU FCE 26858 SUR LES TREMBLEMENTS ET L'HYPOTHERMIE INDUITS PAR L'OXOTREMORINE 1 mg/kg IP CHEZ LA SOURIS

Protocole experimental

L'étude est réalisée sur des souris mâles d'un poids moyen de 35 grammes. Le FCE 26858 est étudié à la dose de 0,25 mmole/kg, administré en intrapéritonéale en même temps que l'oxotrémorine. L'oxotrémorine à la dose de 1 mg/kg (base) est injectée en intrapéritonéale. Un lot recevant du soluté salé isotonique à 0,9 % en ip en même temps que l'oxotrémorine est réalisé.

Avant l'injection et 30 minutes après l'injection d'oxotrémorine, la température rectale des animaux est mesurée. Trente minutes après l'injection d'oxotrémorine, l'état comportemental est observé et côté selon un barème variant de 0 à 5 selon l'intensité des symptomes.

**Résultats**

A cette dose, le FCE 26858 antagonise nettement les tremblements, la rigidité et l'akinésie sans modifier de façon nette les signes périphériques induits par l'oxotrémorine (salivation, lacrimation, diarrhée). De plus la diminution de température rectale est significativement moins importante que celle du lot témoin, confirmant

qu'à cette dose le FCE 26858 s'oppose aux effets centraux de l'oxotrémorine (figure 6).

| COTATION MOYENNE DE LA RIGIDITE, AKINESIE ET TREMBLEMENTS | | |
|---|---|---|
| TEMOIN | 4,3 ± 0,28 | |
| FCE 26858 | 2,7 ± 0,28 | -37,2 % ** |

**Conclusion**

Ces résultats suggèrent l'utilisation du FCE 26858 dans le traitement symptomatique de la maladie de Parkinson.

3) ACTIVITE ANTIDEPRESSIVE

Le dysfonctionnement du système dopaminergique central apparaît de plus en plus important dans certains états dépressifs (Willner P. et al. Dopamine, depression and anti-depressant drugs, "The mesolimbic dopamine system : from Motivation to Action", ed. P. Willner and J. Scheel-Krügger, p. 387, 1991).

Ainsi certains antidépresseurs tels l'AMINEPTINE ou la NOMIFENSINE agissent en inhibant la recapture de la dopamine favorisant ainsi son action synaptique (Waldmener P.C., J. Pharm. Pharmacol. Nr 34 : 391-394, 1982). Or la figure 5 montre que le FCE 26858 provoque une libération de dopamine sans en altérer sa synthèse et que la diminution du DOPAC (métabolite intrasynaptosomale) laisse supposer que le FCE 26858 inhibe la recapture de la dopamine. Ainsi le FCE 26858 pourrait rétablir une fonction dopaminergique altérée dans certains états dépressifs.

D'autre part , le rôle du système sérotoninergique dans certains états dépressifs est clairement établi et des études cliniques ont montré que des inhibiteurs de la recapture de la sérotonine (5-HT), favorisant ainsi sa transmission, sont des antidépresseurs actifs (Martin P. et al., Psychopharmacology Nr 101 : 406-407, 1990).

Ainsi l'activité antidépressive du FCE 26858 pourrait être renforcée par son action facilitatrice de la transmission sérotoninérgique puisque son administration provoque une augmentation du métabolite de la 5-HT, l'acide 5-hydroxyndole acétique (5-HIAA) sans diminuer le taux de la 5-HT (figure 5bis).

Nous avons aussi testé le FCE 26858 dans un modèle animal de la dépression le "Behavorial despair" ou test de la nage forcée (Porsolt R. et al. Pharmacological models of depression, p. 137, in "Animal models in psychopharmacology advances in pharmacological sciences, 1990).

ETUDE DU FCE 26858 SUR LE TEST DE LA NAGE FORCEE

Protocole expérimental

Les souris sont forcées à nager individuellement dans un cylindre en plexiglas (hauteur : 26 cm; diamètre 19 cm) contenant 10 cm d'eau maintenue à 24 degrés C ± 1 pendant 6 minutes.

Les animaux sont ensuite essuyés puis réchauffés dans une enceinte chauffée à 30 degrés C. 24 heures plus tard, les souris sont replacées dans le cylindre pendant 6 minutes et la durée totale de l'immobilité est mesurée (on considère que l'animal est immobile lorsqu'il n'effectue que les mouvements nécessaires à sa flottaison).

**Administration des drogues**

Trois lots sont réalisés :
- un lot témoin NaCl 0,9 % ip,
- un lot Imipramine (molécule de référence) 25 mg/kg ip et,
- un lot FCE 26858 0,10 mmole/kg (24 mg/kg ip).

Les traitements sont administrés une heure et cinq heures après la première session de nage forcée et une heure avant le test.

**Résultats**

La figure 7 montre que le FCE 26858 diminue significativement (** $p < 0{,}01$ test de Student) la durée de l'immobilité, comme l'imipramine.

**Conclusion**

Les résultats du FCE 26858 sur le turnover de la DA et de la 5-HT et sur le test de la nage forcée expliquent son utilisation dans le traitement des états dépressions.

4) ACTIVITE ANTIPSYCHOTIOUE

Nous avons testé l'action du FCE 26858 dans le test de l'inhibition latente chez le rat, modèle animal du déficit attentionnel des schizophrènes. Les neuroleptiques ont un effet faciliateur dans ce modèle et Feldon et Weiner (Biol. Psychiatry Nr 29: 635-646, 1991) décrivent l'inhibition latente comme un test permettant de détecter le potentiel antipsychotique de certaines molécules.

ETUDE DU FCE 26858 SUR LE TEST DE L'INHIBITION LATENTE

Protocole expérimental

**Animaux**

Les animaux utilisés sont des rats Sprague-Dawley d'un poids de 250-300 grammes séparés en groupes de 7 animaux.

**Appareillage**

L'appareillage utilisé est une cage basculante autour d'un axe entre deux compartiments identiques séparés par une cloison opaque comportant un trou de fuite.

Le plancher est composé de barreaux électrifiables de 2 cm de diamètre espacés de 1 cm.

La décharge électrique nociceptive (1 mA), précédée d'un stimulus sonore, est programmée par un Automatic reflex conditionner UGO BASILE.

La cage de conditionnement est placée dans une enceinte insonorisée.

Le protocole de l'inhibition latente est constitué de deux étages : la préexposition (PE) et le test d'évitement.

Préexposition :

Chaque animal est placé dans la cage de conditionnement et reçoit 10 ou 50 stimuli sonores de 6,5 secondes selon un intervalle entre les stimuli variant de 20 à 36 secondes.

Les animaux non préexposés (NPE) sont mis dans la cage pendant le même temps mais ne reçoivent aucun stimulus.

- Test d'évitement :

24 heures après la préexposition, chaque animal est placé dans la cage de conditionnement et reçoit 60 essais d'évitement selon un intervalle entre les essais variant de 20 à 36 secondes. Chaque essai d'évitement démarre avec la présentation du stimulus sonore pendant 3 secondes suivi d'un stimulus électrique de 3,5 secondes. Le stimulus sonore demeure pendant la durée du stimulus électrique. Si l'animal passe dans le compartiment opposé pendant les 3 premières secondes du stimulus sonore, le choc électrique n'est pas délivré et l'évitement est comptabilisé.

Les 60 essais sont divisés en blocs de 12 essais et les résultats sont exprimés en pourcentage d'évitement dans chaque bloc.

La comparaison des groupes est effectuée à l'aide de l'analyse de variance avec * $p < 0{,}05$ et ** $p < 0{,}01$.

**Drogues**

Le NaCl 0,9 %, le sulpiride 50 mg/kg (neuroleptique de référence) et le FCE 26858 0,10 mmole/kg (24

mg/kg ip) sont administrés une heure avant la préexposition et le test.

**Résultats**

- La figure 8 montre que la préexposition des animaux à 50 stimuli sonores provoque dans le groupe PE une diminution significative du pourcentage d'évitement caractérisant l'inhibition latente. Par contre, si le nombre de stimuli sonores se limite à 10 lors de la préexposition, cela n'est pas suffisant pour induire une inhibition latente 24 heures après dans le groupe témoin.

Cependant si les animaux reçoivent du sulpiride 50 mg/kg, une inhibition latente significative apparaît dans le groupe préexposé avec 10 stimuli seulement (figure 9).

De même le FCE 26858 24 mg/kg ip favorise l'apparition de l'inhibition latente chez les animaux préexposés à 10 stimuli sonores (figure 10).

**Conclusion**

Les résultats montrent que comme le sulpiride, neuroleptique atypique, le FCE 26858 favorise l'apparition de l'inhibition latente, expliquant ainsi le potentiel antipsychotique de cette molécule.

<u>CONCLUSION GENERALE</u>

Les résultats expérimentaux présentés ici montrent que les produits selon l'invention peuvent être utilisés en thérapeutique humaine dans diverses affections neurologiques et psychiatriques :

- traitement des troubles mnésiques associés à l'âge avec traumatismes crâniens et aux maladies dégénératives aux faibles doses,
- traitement symptomatique de la maladie de Parkinson,
- traitement des états dépressifs,
- traitement des psychoses.

Ces produits peuvent être administrés par voie orale ou par voie parentérale, et possèdent un coefficient thérapeutique très favorable car la dose léthale 50 du FCE 26858 est de 380 mg/kg ip chez la souris.

TABLEAU 1

Structures des composés

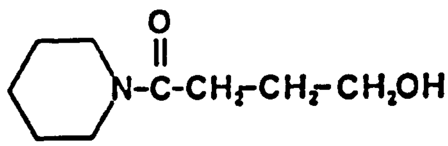

26099

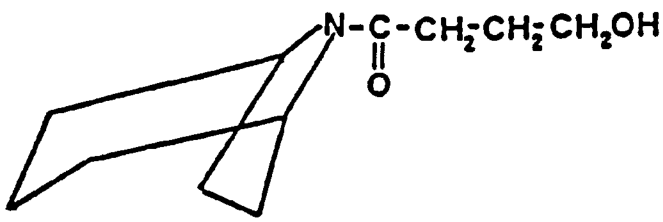

26630

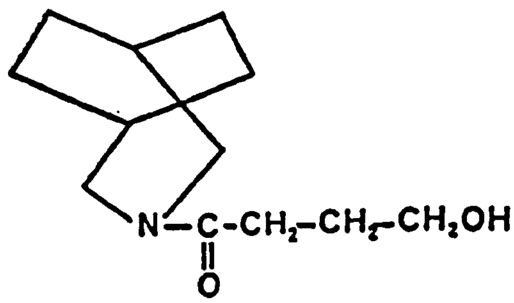

26857

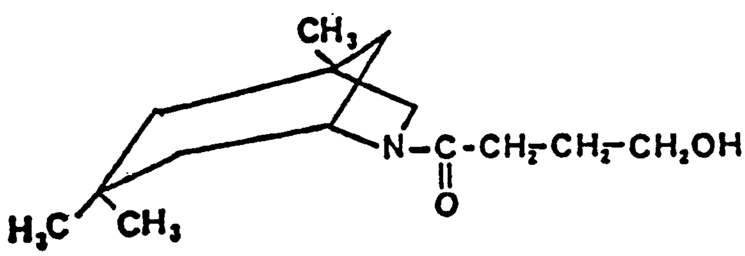

26858

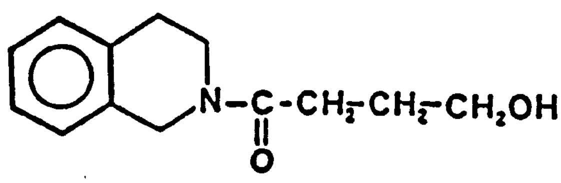

27407

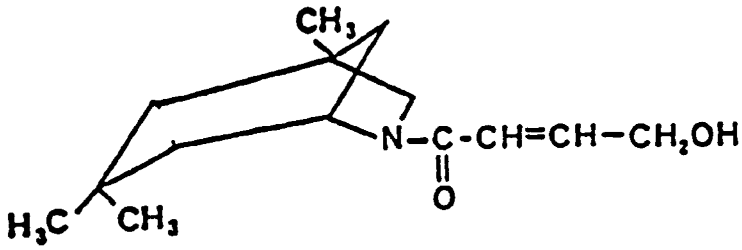

27363

**Revendications**

1. Dérivés cycliques représentés par la formule générale

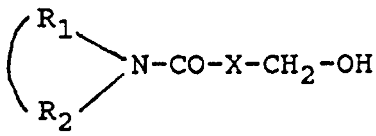

(I)

dans laquelle le système cyclique

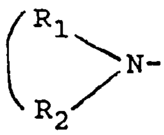

représente, nor-tropane, 3-azabicyclo [3 , 2, 2] nonane, 6-azabicyclo [3,2,1]octane, ou tétrahydro-1,2,3,4-isoquinoline, éventuellement substitué par un ou plusieurs groupes alkyle, et
X représente le résidu $CH_2$-$CH_2$ ou CH=CH (trans)

2. Agent thérapeutique pour le traitement des affections neurologiques et psychiatriques contenant une quantité efficace d'un dérivé cyclique représenté par la formule générale

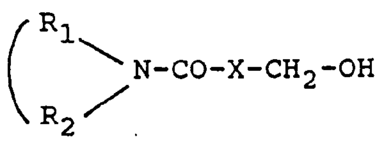

(I)

dans laquelle le système cyclique

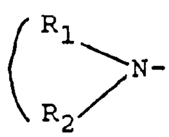

représente, pipéridine, nor-tropane, 3-azabicyclo[3,2,2]nonane, 6-azabicyclo [3,2,1]octane, ou tétrahydro-1,2,3,4-isoquinoline, éventuellement substitué par un ou plusieurs groupes alkyle, et
X représente le résidu $CH_2$-$CH_2$ ou CH=CH (trans)

3. Utilisation d'un dérivé cyclique représenté par la formule générale

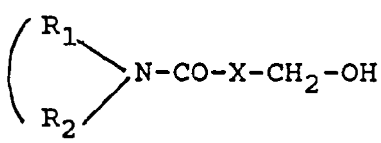

(I)

dans laquelle le système cyclique

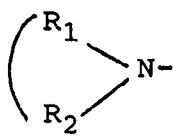

représente, pipéridine, nor-tropane, 3-azabicyclo[3,2,2]nonane, 6-azabicyclo [3,2,1]octane, ou tétrahydro-1,2,3,4-isoquinoline, éventuellement substitué par un ou plusieurs groupes alkyle, et
X représente'le résidu $CH_2$-$CH_2$ ou CH=CH (trans) pour la préparation d'un produit pharmaceutique destiné au traitement des affections neurologiques et psychiatriques.

ETUDE DU FCE 26353 SUR
L'AMNESIE INDUITE PAR LA SCOPOLAMINE SUR
UN TEST D'EVITEMENT PASSIF.

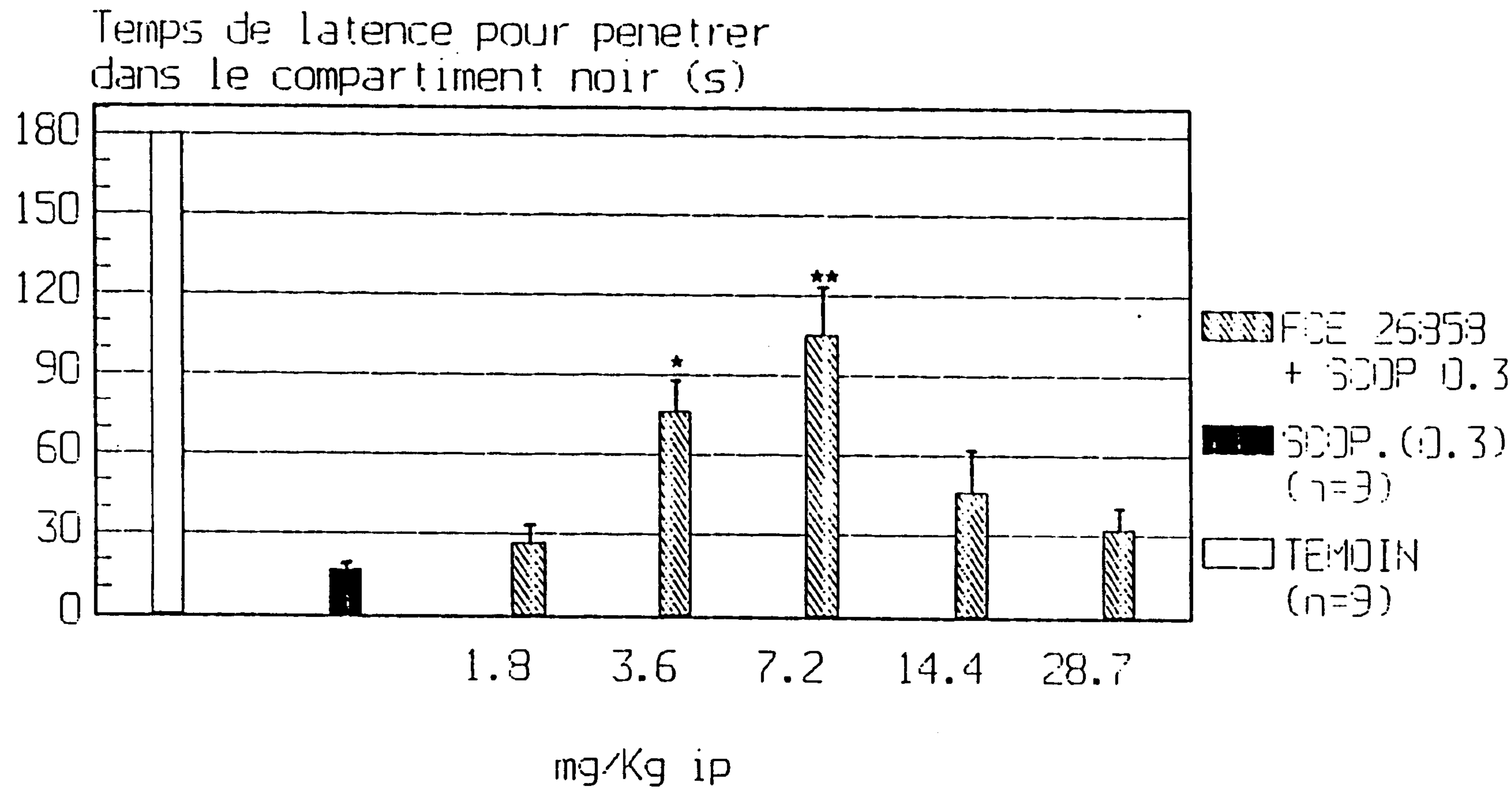


FIGURE 1

ETUDE DE L'ANIRACETAM SUR L'AMNESIE INDUITE PAR LA SCOPOLAMINE SUR UN TEST D'EVITEMENT PASSIF.

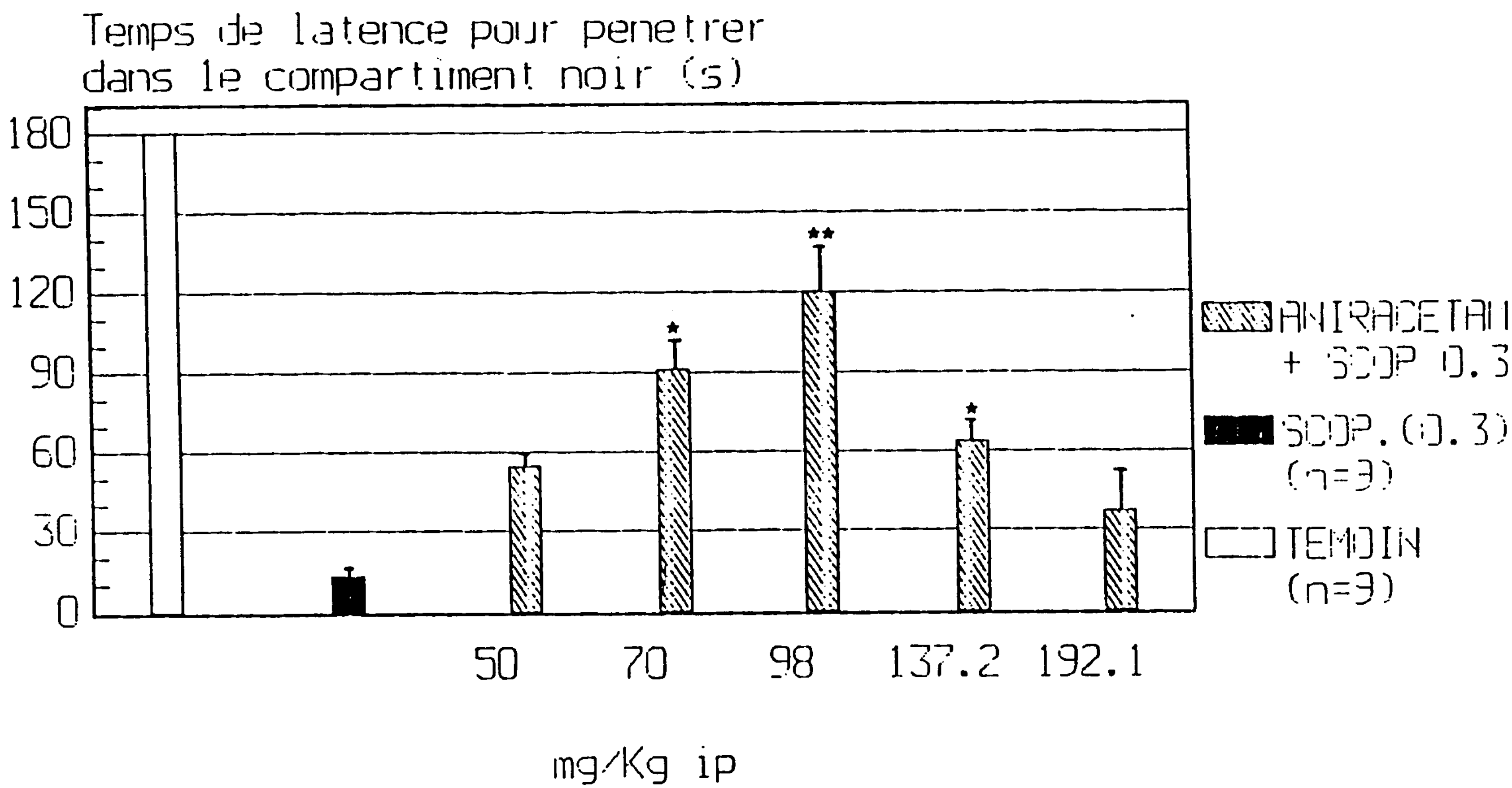


FIGURE 2

ETUDE DU FCE 26858 (mmole/Kg ip) ET DE L'ANIRACETAM SUR UN TEST D'EVITEMENT ACTIF : "WALL JUMP TEST"

Evolution du score d'apprentissage

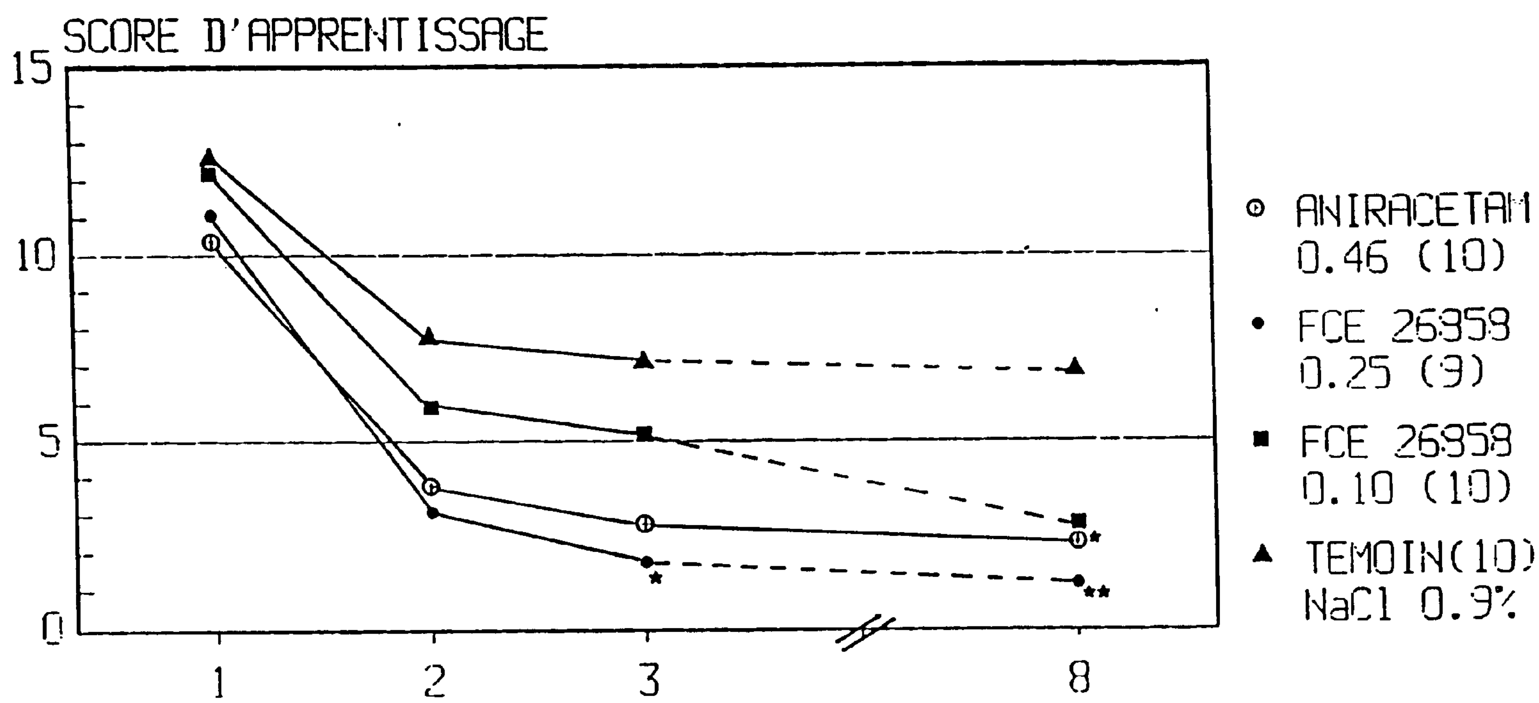


FIGURE 3

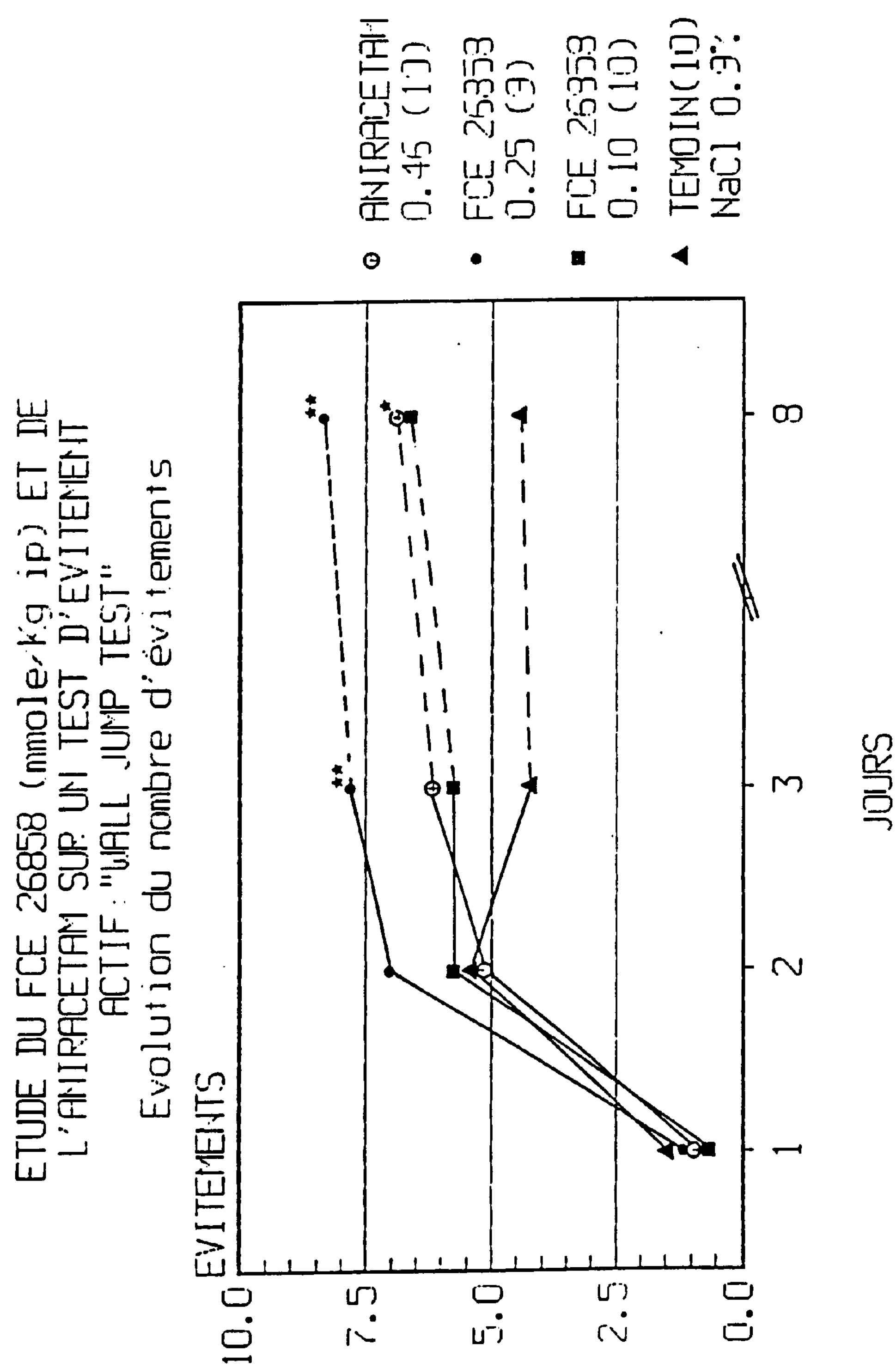
ETUDE DU FCE 26858 (mmole/kg ip) ET DE L'ANIRACETAM SUR UN TEST D'EVITEMENT ACTIF : "WALL JUMP TEST"
Evolution du nombre d'évitements
EVITEMENTS
10.0
7.5
5.0
2.5
0.0
1
2
3
8
JOURS
**
*
ANIRACETAM 0.46 (10)
FCE 26858 0.25 (9)
FCE 26858 0.10 (10)
TEMOIN(10) NaCl 0.9%
FIGURE 4

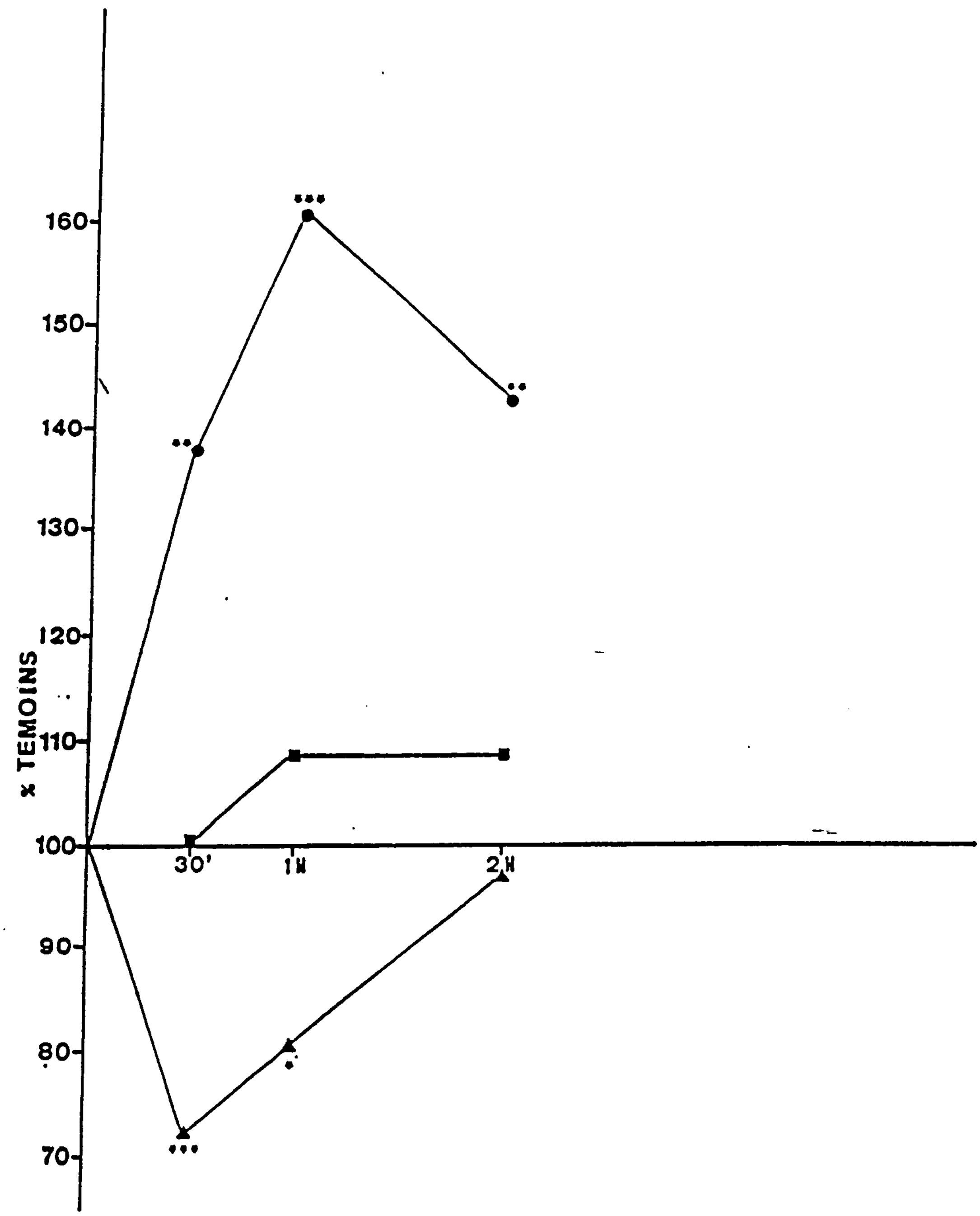


VARIATION DES TAUX DE DA(■).DE DOPAC(▲)
ET DE HVA(•) DANS LE STRIATUM DE CERVEAU
DE SOURIS SACRIFIEES 30 ,60 ET 120
MINUTES APRES FCE 26858 0.25mmole/kg IP.

FIGURE 5

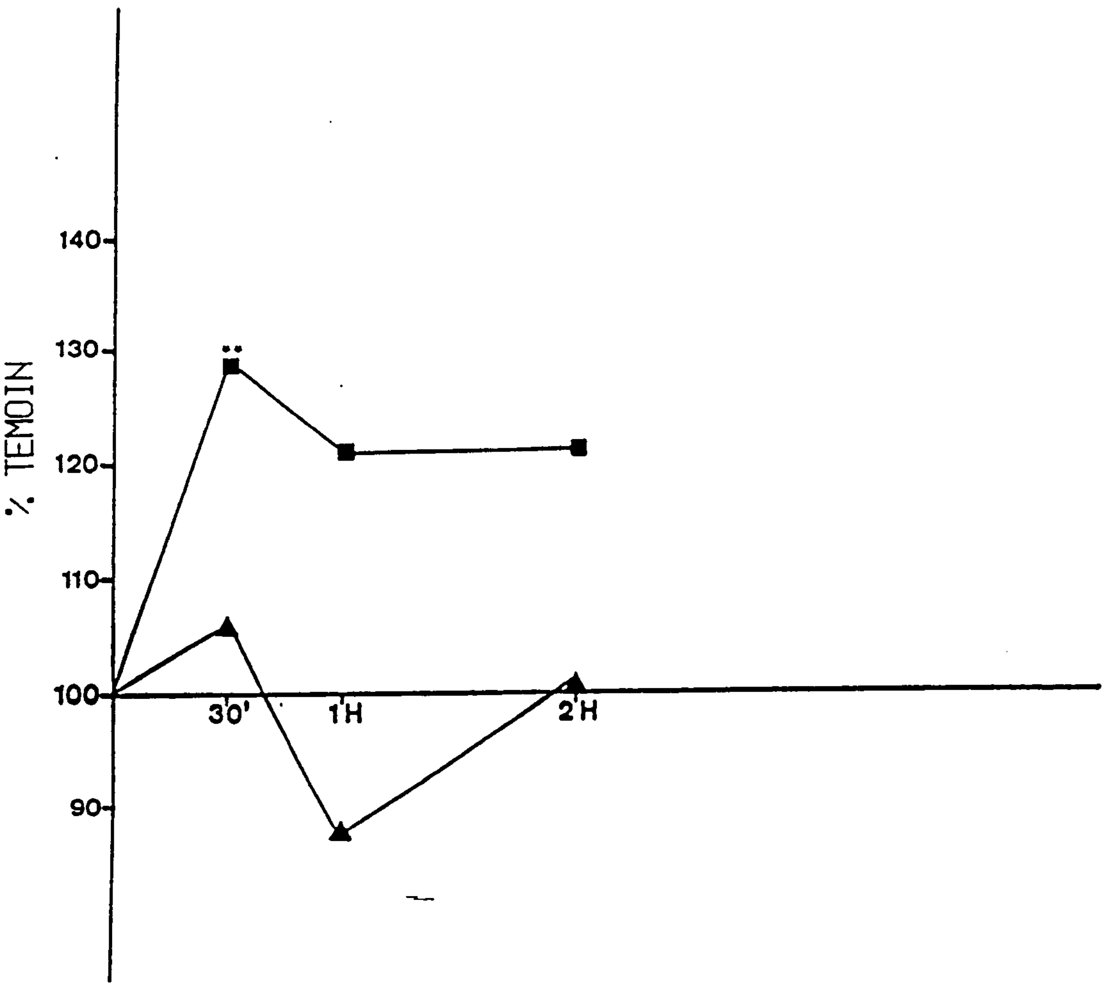


VARIATION DES TAUX DE 5-HT(▲) ET DE 5-HIAA(■) MESURES DANS LE STRIATUM DE CERVEAU DE SOURIS 30,60 ET 120 MINUTES APRES FCE 26858 0.25mmole/kg ip.

FIG. 5 BIS

ETUDE DU FCE 26858(mmole/kg) SUR LA
DIMINUTION DE TEMPERATURE RECTALE
INDUITE PAR L'OXOTREMORINE 1 mg/Kg i.p.

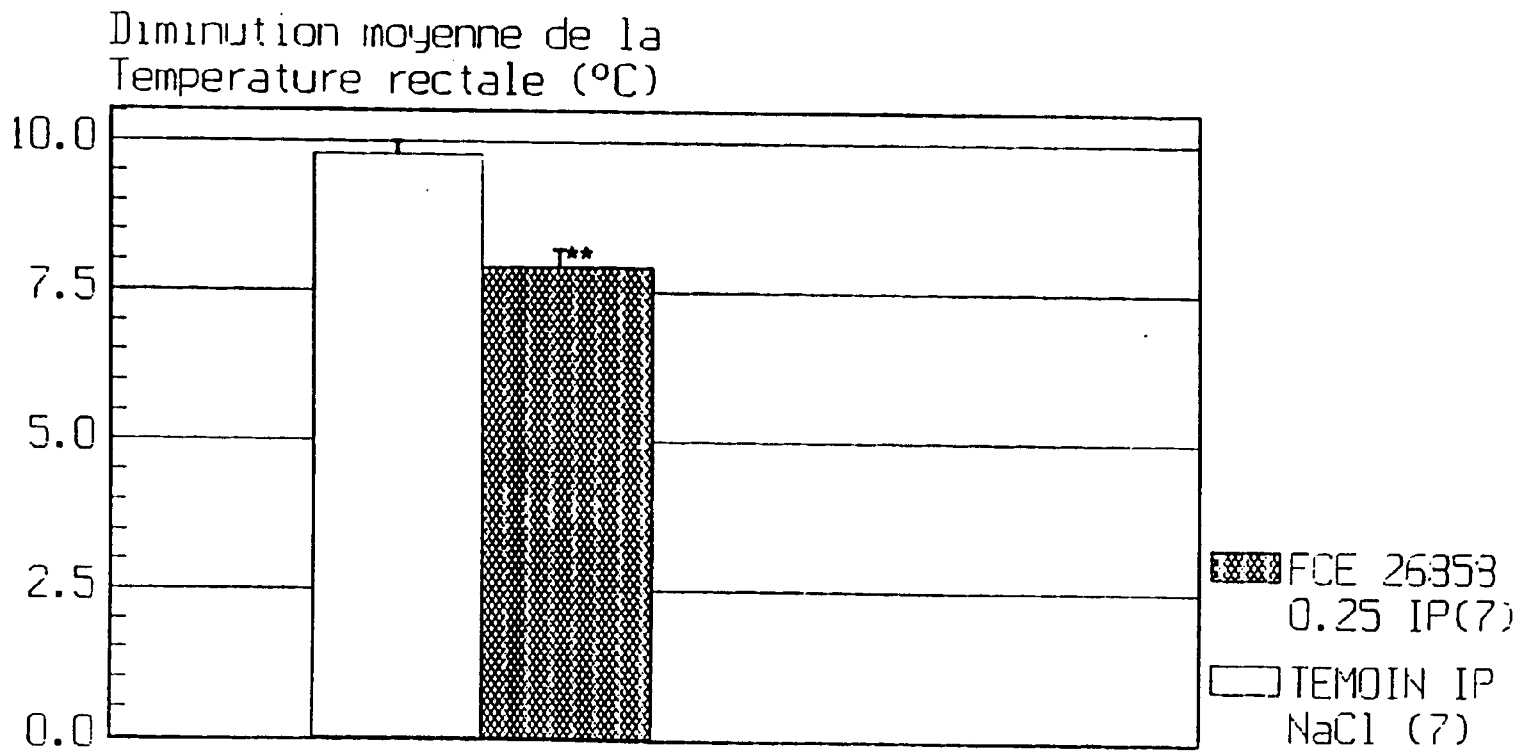


FIGURE 6

ETUDE DU FCE 26858 ET DE L'IMIPRAMINE SUR LA DUREE DE L'IMMOBILITE DANS LE TEST DE LA NAGE FORCEE ("Behavioral Despair") CHEZ LA SOURIS.

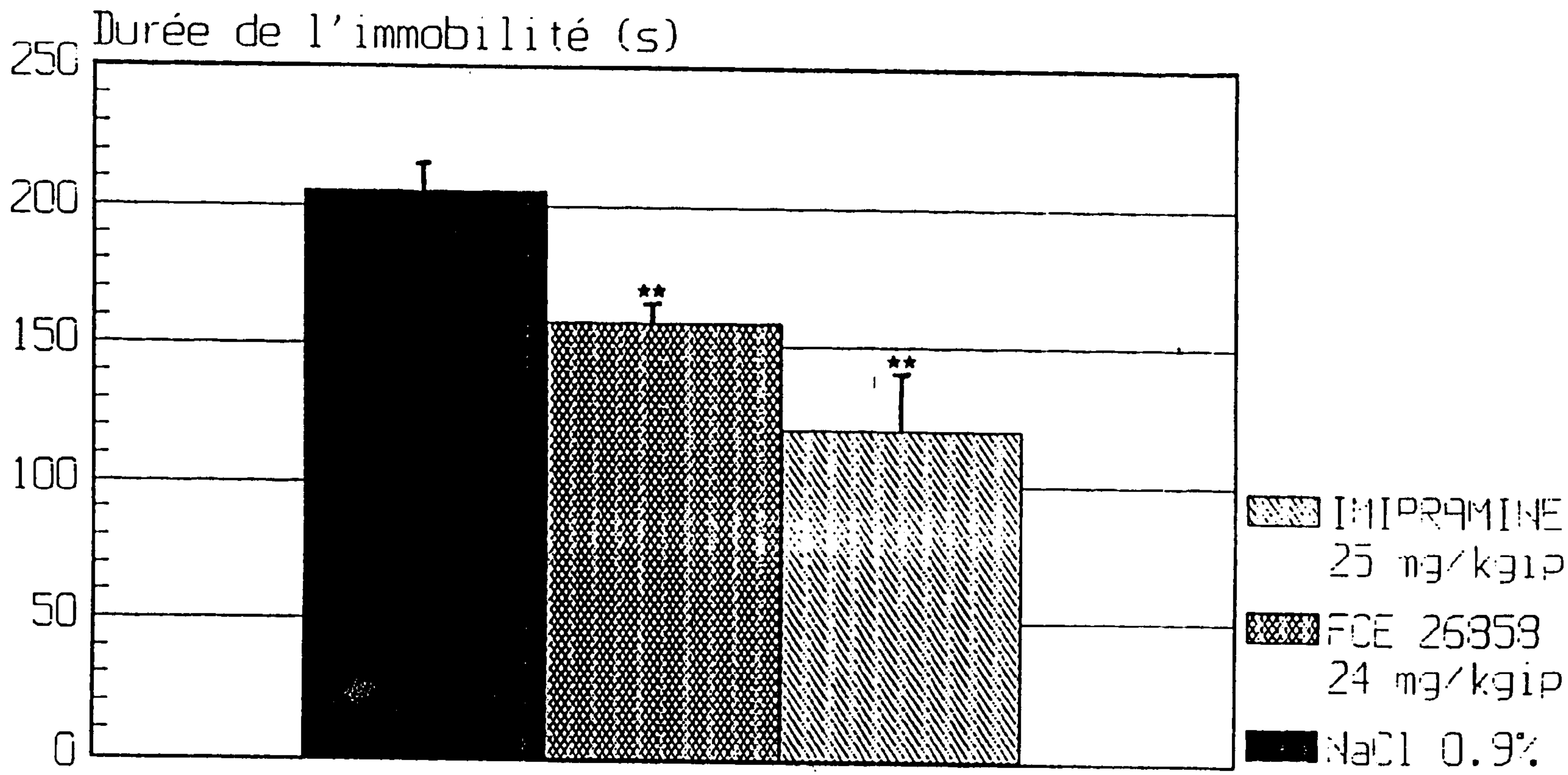


FIGURE 7

TEST DE L'INHIBITION LATENTE
(10 stimuli conditionnes)

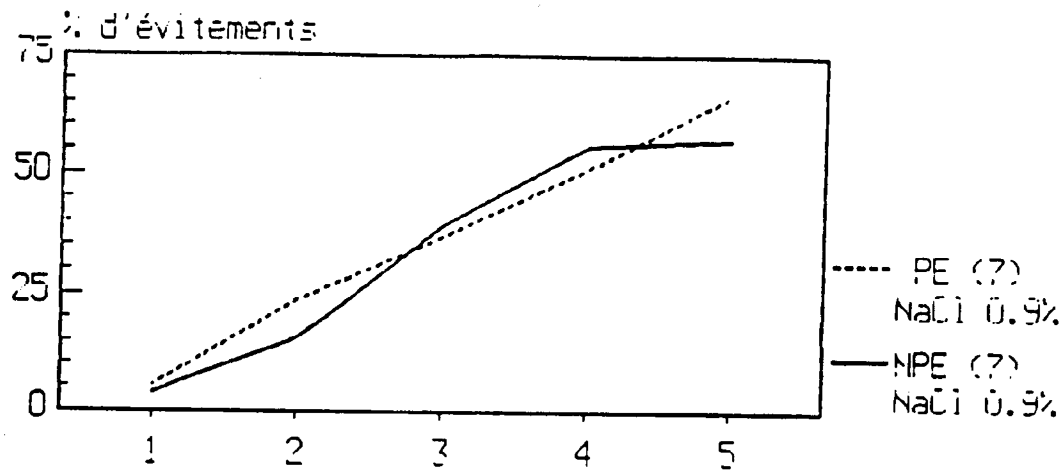


Blocs
ANOVA PE-NPE F=0.074 NS ESM=5.60

TEST DE L'INHIBITION LATENTE
(60 stimuli conditionnes)

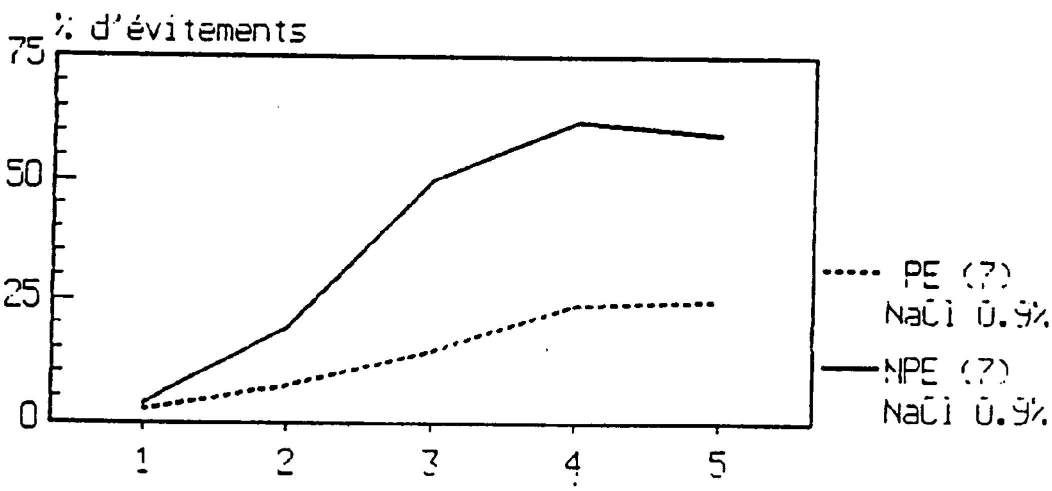


Blocs
ANOVA PE-NPE F=11.21 S** ESM=5.10

FIGURE 8

TEST DE L'INHIBITION LATENTE
(10 stimuli conditionnes)

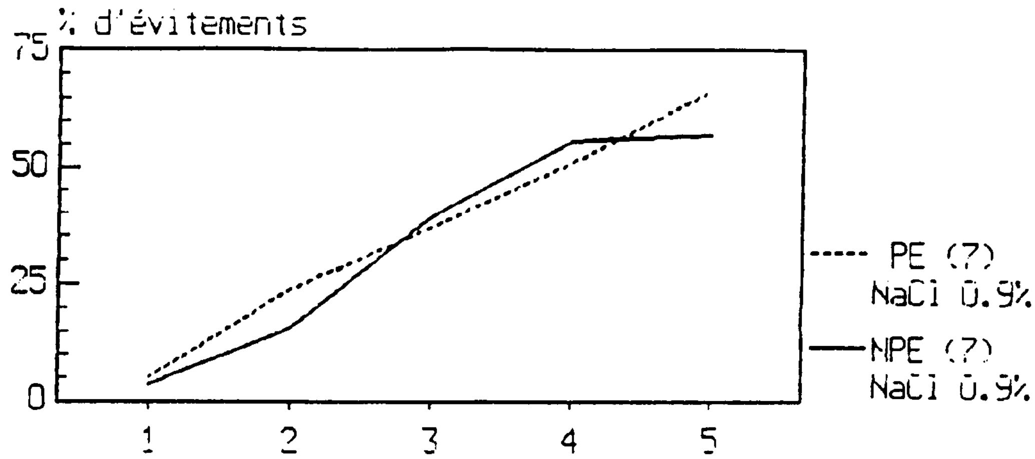


Blocs
ANOVA:PE-NPE F=0.074 NS ESM=5.60

TEST DE L'INHIBITION LATENTE
ETUDE DU SULPIRIDE 50 mg/Kg i.p.
(10 stimuli conditionnés)

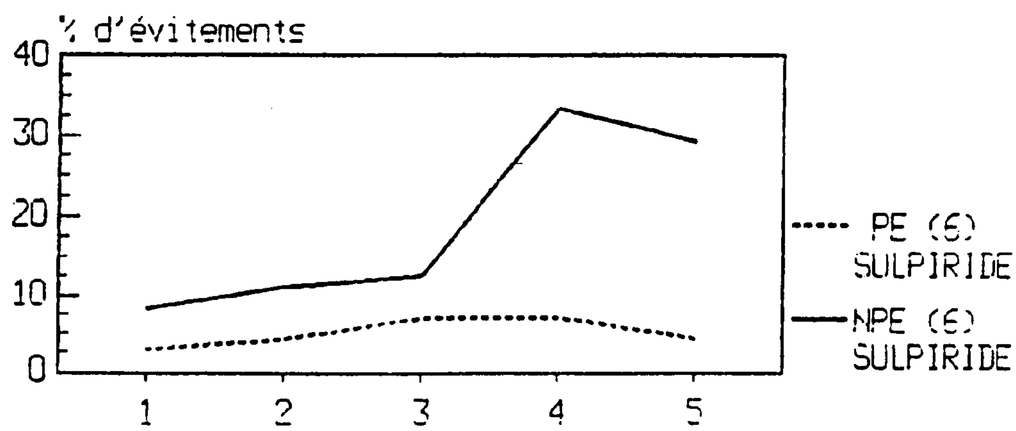


Blocs
ANOVA:PE-NPE F=12.38 S*** ESM=2.79

FIGURE 9

TEST DE L'INHIBITION LATENTE
(10 stimuli conditionnes)

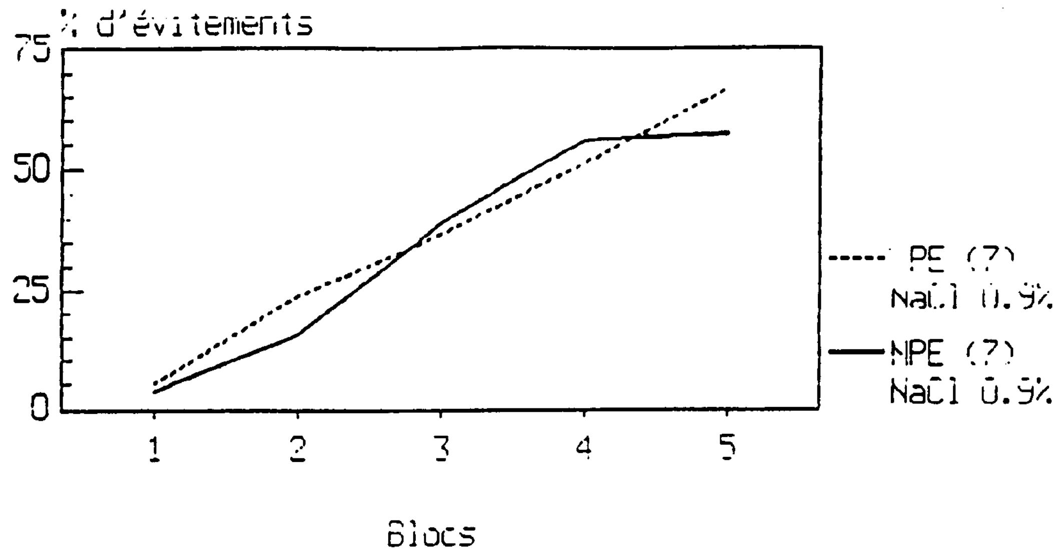


ANOVA:PE-NPE F=0.074 NS ESM=5.60

TEST DE L'INHIBITION LATENTE
(10 stimuli conditionnes)
ETUDE DU FCE 26858 0.10mmole/Kg ip

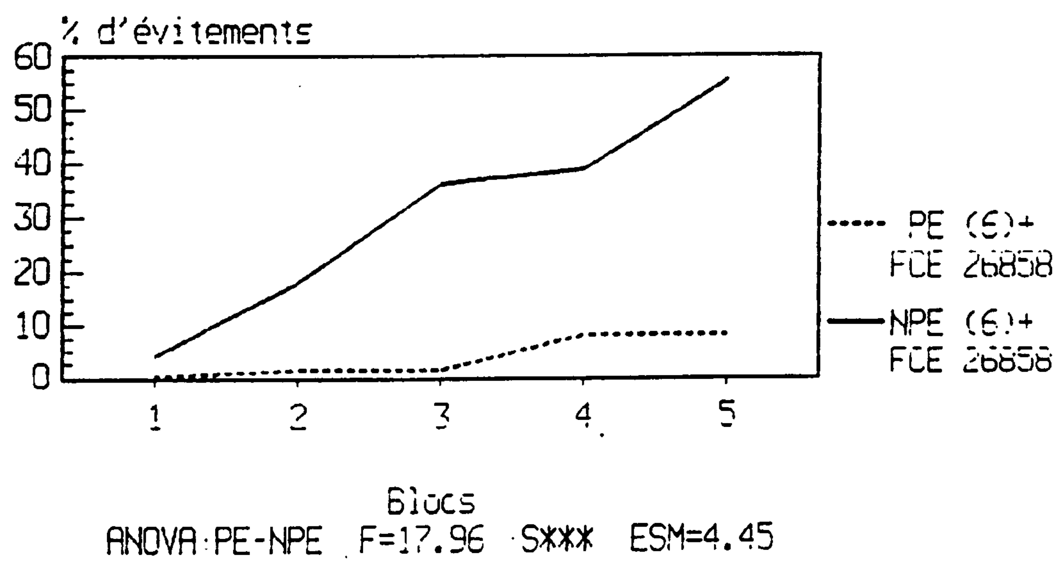


ANOVA:PE-NPE F=17.96 S*** ESM=4.45

FIGURE 10